# EUROPÄISCHE PATENTSCHRIFT

(11) **EP 4 014 845 B1**
(45) Veröffentlichungstag und Bekanntmachung des Hinweises auf die Patenterteilung: **15.05.2024**
(21) Anmeldenummer: 21211192.6
(22) Anmeldetag: 30.11.2021
(51) Int. Cl.: A61B 5/00

(54) **AUSGABEEINRICHTUNG ZUM AUSGEBEN EINES ZEITLICHEN MESSWERTVERLAUFS**
OUTPUT DEVICE FOR OUTPUTTING A CHANGE IN A MEASUREMENT OVER TIME
DISPOSITIF DE SORTIE PERMETTANT DE SORTIR UN PROCESSUS TEMPORAIRE DE MESURE

(30) Priorität: 02.12.2020 DE 102020131947
(43) Veröffentlichungstag der Anmeldung: 22.06.2022
(73) Patentinhaber: Drägerwerk AG & Co. KGaA, 23558 Lübeck (DE)
(72) Erfinder: Gömann, Michael, 23558 Lübeck (DE); Weber-Lehn, Christian, 23558 Lübeck (DE)

(56) Entgegenhaltungen:
- DE-A1-102005 006 657
- US-A1- 2010 249 549
- US-A1- 2018 310 822
- US-A1- 2019 090 789
- US-A1- 2020 043 606

## Beschreibung

Die Erfindung betrifft eine Ausgabeeinrichtung zum Ausgeben eines zeitlichen Messwertverlaufs eines medizinischen Messwerts. Weiterhin betrifft die Erfindung ein Ausgabesystem zum Ausgeben eines zeitlichen Messwertverlaufs eines medizinischen Messwerts, ein Verfahren zum Ausgeben eines solchen zeitlichen Messwertverlaufs und ein Computerprogramm mit einem Programmcode zur Durchführung eines solchen Verfahrens.

Es ist allgemein bekannt, Messwertverläufe im medizinischen Kontext auszugeben, um die physiologische Entwicklung eines Patienten während seiner Behandlung zu erkennen und für die weitere Behandlung berücksichtigen zu können. Weiterhin ist bekannt, dass man manuell oder automatisiert Kennzahlen aus einem Messwertverlauf entnimmt. Eine solche Kennzahl kann bei der klinischen Beurteilung des aktuellen Messwerts des Messwertverlaufs helfen. Weiterhin kann dadurch schnell und sicher ein aktueller Zustand des Patienten erfasst werden.

Bekanntermaßen kann die Ausgabe des Messwertverlaufs beabstandet von dem eigentlichen Medizingerät erfolgen. Beispielsweise kann das Medizingerät über ein Netzwerk mit einem entsprechenden Ausgabegerät verbunden sein. So beschreibt die deutsche Patentanmeldung DE 10 2019 003 995 eine Anzeigeeinheit, die mit einem Medizingerät verbunden ist, um als separates Gerät entsprechende Messdaten graphisch auszugeben. US 2019/090789 A1, US 2010/249549 A1 und US 2018/310822 A1 sind ebenfalls beispielhafte Systeme grafischer Darstellungen von Messdaten. U

Aufgabe der vorliegenden Erfindung ist es, eine verbesserte Ausgabeeinrichtung, insbesondere eine Ausgabeeinrichtung mit besonders einfach zu erfassender Signalqualität, bereitzustellen.

Gemäß einem ersten Aspekt der Erfindung wird zur Lösung dieser Aufgabe eine Ausgabeeinrichtung zum Ausgeben eines zeitlichen Messwertverlaufs eines medizinischen Messwerts vorgeschlagen, mit einer Empfangseinheit, einer Ausgabesteuerungseinheit und einer Displayeinheit.

Die Empfangseinheit ist ausgebildet, Sensordaten und Signalgütedaten über mindestens ein Empfangssignal zu empfangen und zur Weiterverarbeitung durch die Ausgabeeinrichtung bereitzustellen, wobei die Sensordaten den zeitlichen Messwertverlauf des medizinischen Messwerts indizieren, und wobei die Signalgütedaten einen dem zeitlichen Messwertverlauf zugeordneten zeitlichen Signalgüteverlauf indizieren.

Die Ausgabesteuerungseinheit ist ausgebildet, basierend auf den Sensordaten und den Signalgütedaten ein auszugebendes Diagramm mit einer Messwertverlaufskurve zu bestimmen und über ein Ausgabesignal bereitzustellen, wobei die Messwertverlaufskurve den zeitlichen Messwertverlauf und eine Verlaufskurvenaufspreizung des Messwertverlaufs anzeigt, und wobei die Messwertverlaufskurve durch ihre Lage und Struktur innerhalb des auszugebenden Diagramms den zeitlichen Messwertverlauf des medizinischen Messwerts indiziert. Dabei ist die jeweils einem Zeitpunkt der Messwertverlaufskurve zugeordnete Verlaufskurvenaufspreizung ein Maß für die zu diesem Zeitpunkt der Messwertverlaufskurve entsprechend der Signalgütedaten vorliegende Signalgüte.

Die Displayeinheit ist ausgebildet, das Ausgabesignal zu empfangen und auf einem Display der Displayeinheit das auszugebende Diagramm mit der Messwertverlaufskurve und der entsprechenden Verlaufskurvenaufspreizung graphisch auszugeben, wobei die Verlaufskurvenaufspreizung als entsprechende Aufspreizung des zeitlichen Messwertverlaufs dargestellt ist.

Im Rahmen der Erfindung wurde erkannt, dass zusammen mit dem Messwertverlauf weitere Informationen zum Bewerten dieses Messwertverlaufs ausgegeben werden können. So wird zusammen mit dem Messwertverlauf eine Ausgabe einer Aufspreizung des Messwertverlaufs vorgeschlagen, um unmittelbar mit dem Messwertverlauf auch eine Information bezüglich der aktuell vorliegenden Signalgüte an einen Nutzer der Ausgabeeinrichtung auszugeben. Insbesondere wird hierdurch vorteilhaft die Ausgabe von zwei verschiedenen visuellen Bereichen für die Ausgabe dieser Information auf einem Display vermieden.

Es ist zwar bekannt, eine Zahl oder ein gesondertes grafisches Element, wie etwa einen Balken anzuzeigen, um eine aktuelle Signalgüte darzustellen. Demgegenüber hat die erfindungsgemäße Lösung jedoch den Vorteil, dass neben der Erfassung des aktuell Messwertverlaufs keine zusätzliche kognitive Aktivität erforderlich ist, um unmittelbar die Verlaufskurvenaufspreizung und damit eine Information bezüglich der aktuell vorliegenden Signalgüte wahrzunehmen.

Weiterhin erlaubt die Darstellung der Verlaufskurvenaufspreizung für den Messwertverlauf auch eine Einschätzung der Genauigkeit von dargestellten früheren Werten, bei denen möglicherweise eine besonders niedrige oder eine besonders höhere Signalgüte vorlag. Somit unterstützt die erfindungsgemäße Ausgabeeinrichtung eine besonders zuverlässige Auswertung der dargestellten medizinischen Messwerte.

Die direkte Zuordnung von Sensordaten und Signalgütedaten ermöglicht die Darstellung einer zeitlichen Abfolge von Aufspreizungen des Messwertverlaufs. Hierdurch wird unmittelbar eine sich eventuell einstelle Tendenz zu besonders hoher oder besonders niedriger Signalgüte erkennbar, sodass Veränderungen einer Kommunikationsverbindung zu dem entsprechenden medizinischen System unmittelbar erkannt und bei der Auswertung der entsprechenden Messwerte berücksichtigt werden können. Zudem können physische Änderungen bei der Kommunikationsverbindung angeregt werden, wie etwa eine Umstellung von kabelloser Verbindung zu kabelbasierter Verbindung und/oder eine Veränderung des aktuellen Standortes der Ausgabeeinrichtung.

Dass Sensordaten und Signalgütedaten über mindestens ein Empfangssignal empfangen werden, bedeutet dass sie innerhalb eines einzelnen Signals, durch zwei getrennte Signale und/oder durch eine Mehrzahl von Signalen, wie etwa durch eine Abfolge von separaten Signalen erfindungsgemäß empfangen werden können.

Das auszugebende Diagramm erlaubt erfindungsgemäß aufgrund seiner Struktur eine Aussage über eine quantitative Eigenschaft des aktuell vorliegenden Messwerts. Hierfür kann das Diagramm ein Koordinatensystem oder vergleichbare grafische Mittel zum Veranschaulichen einer Größe oder eines aktuellen Bereichs des aktuell vorliegenden Messwerts umfassen.

Eine Aufspreizung ist eine visuelle Veranschaulichung eines Bereichs, der breiter ist als ein bloßer Punkt oder eine bloße Linie, die den aktuell vorliegenden Messwert und/oder den aktuell vorliegenden Messwertverlauf anzeigt.

Die Messwertverlaufskurve kann den Messwertverlauf kontinuierlich oder in Form von diskreten Messwerten, wie etwa diskreten Punkten innerhalb des Diagramms, anzeigen.

Die Einheiten der erfindungsgemäßen Ausgabeeinrichtung können zumindest teilweise beabstandet voneinander, insbesondere teilweise in separaten Gehäusen, vorliegen. Vorzugsweise sind die Einheiten in einem gemeinsamen Gehäuse angeordnet. Insbesondere werden die Einheiten vorzugsweise von einem gemeinsamen Prozessor ausgeführt wobei sie dabei zumindest auf Softwareebene voneinander getrennt sind.

Nachfolgend werden bevorzugte Ausführungsformen der erfindungsgemäßen Ausgabeeinrichtung beschrieben.

In einer vorteilhaften Ausführungsform ist die Aufspreizung eine symmetrische Aufspreizung des Messwertverlaufs um einen durch die Sensordaten indizierten Messwert als Mittelpunkt der Aufspreizung. In dieser Ausführungsform führt eine geringe Signalgüte zu der Annahme eines symmetrischen Fehlers um den auszugebenden Messwert. Hierdurch kann besonders schnell die Signalgüte erfasst werden, da durch den Nutzer nicht zwei Messwertbereiche um den eigentlichen Messwert miteinander verglichen werden müssen. Das Vorsehen einer symmetrischen Aufspreizung kann vorteilhaft zu einer geringeren Verrechnungsdauer durch die Ausgabeeinrichtung führen als für den Fall, dass zwei verschiedene Aufspreizungsbereiche bestimmt werden müssen.

In einer besonders bevorzugten Ausführungsform sind den Messwertverlauf indizierende Bereiche der Messwertverlaufskurve kontrastreicher dargestellt als die entsprechende Aufspreizung. Hierdurch kann der Nutzer der erfindungsgemäßen Ausgabeeinrichtung besonders schnell und zuverlässig den jeweiligen Messwert innerhalb der Messwertverlaufskurve erkennen, ohne die Struktur der Messwertverlaufskurve zeitaufwendig analysieren zu müssen. Besonders bevorzugt wird der Messwertverlauf einfarbig, beispielsweise schwarz bei einem weißen Hintergrund und weiß bei einem dunklen Hintergrund, dargestellt. Vorzugsweise weist die Aufspreizung hierbei ausgehend von dem Messwertverlauf verschiedene Kontraststärken auf. So ist ein äußerer Bereich der Aufspreizung vorzugsweise nahezu in der Farbe des Hintergrundes der Darstellung dargestellt.

In einer vorteilhaften Ausführungsform ist die Aufspreizung als eine kontinuierlich verlaufende Aufspreizung um die den Messwertverlauf indizierenden Bereiche der Messwertverlaufskurve dargestellt. Eine kontinuierlich verlaufende Aufspreizung ist vorzugsweise eine Aufspreizung, deren äußerer Bereich einen stetigen, insbesondere stetig differenzierbaren, Rand aufweist, also insbesondere einen Rand ohne Sprungstellen oder dergleichen. Hierdurch ist jedem Zeitpunkt innerhalb des Messwertverlaufs eindeutig eine Signalgüte zugeordnet. In einer alternativen Ausführungsform ist die Aufspreizung als eine diskontinuierliche Aufspreizung, insbesondere als eine diskret für einzelne Messwerte zu einzelnen Zeitpunkten dargestellte Aufspreizung, vorgesehen.

Besonders bevorzugt ist die Aufspreizung umgekehrt proportional zu der zu diesem Zeitpunkt entsprechend der Signalgütedaten vorliegenden Signalgüte. In dieser Ausführungsform ist die Aufspreizung besonders verständlich, da eine hohe Signalgüte zu einer geringen Aufspreizung und somit zu einem besonders schmalen Aufspreizungsbereich um den gemessenen Messwert des Messwertverlaufs herum führt. So kann die Aufspreizung intuitiv als möglicher Messfehler interpretiert werden, der dann besonders groß ist, wenn die Signalgüte besonders klein ist. Tatsächlich handelt es sich dabei erfindungsgemäß nicht um einen Messfehler, sondern um einen Signalübertragungsfehler. In einer erfindungsgemäßen Ausführungsform ist die Aufspreizung zudem abhängig von einem vorbestimmten systematischen Messfehler desjenigen Gerätes, welches die Messwerte ermittelt hat. Ein derartiger systematischer Messfehler kann in einem entsprechenden Speichermodul hinterlegt sein. In einer alternativen oder ergänzenden erfindungsgemäßen Ausführungsform wird ein aufgrund einer aktuellen Behandlung eines Patienten bekannter temporärer Messfehler bei der Bestimmung einer Größe der Verlaufskurvenaufspreizung berücksichtigt. Ein derartiger temporärer Messfehler kann beispielsweise durch eine Kreislaufbelastende Aktivität vorliegen.

In einer bevorzugten Ausführungsform ist die Ausgabesteuerungseinheit dazu ausgebildet, bei der Bestimmung der Größe der Verlaufskurvenaufspreizung einen zeitlichen Abstand zu der letzten erfolgten Messung eines entsprechenden medizinischen Messwertes zu berücksichtigen. So kann für eine stark schwankende Größe schon nach wenigen Sekunden ein ermittelter Messwert mit einer erheblichen Ungenauigkeit belastet sein, da dieser Messwert möglicherweise nur noch eine geringe Aussagekraft über den aktuell tatsächlich vorliegenden Messwert hat.

In einer bevorzugten Ausführungsform ist die Ausgabesteuerungseinheit weiter ausgebildet, auf ein Speichermodul mit einer hinterlegten Mehrzahl von graphischen Darstellungen für das auszugebende Diagramm zuzugreifen. Hierdurch ist eine benutzerdefinierte Einstellung der graphischen Darstellung der Messwertverlaufskurve möglich. Die hinterlegte Mehrzahl von graphischen Darstellungen kann verschiedene Farben und/oder Farbkombinationen für das auszugebende Diagramm umfassen. Weiterhin können die verschiedenen graphischen Darstellungen verschiedene Darstellungen für die Verlaufskurvenaufspreizung umfassen.

In einer vorteilhaften Ausführungsform ist die Empfangseinheit weiter ausgebildet, Alarmierungsdaten zu empfangen, wobei die Alarmierungsdaten mindestens eine Alarmierungsgrenze für den medizinischen Messwert indizieren, und wobei die Ausgabesteuerungseinheit weiter ausgebildet ist, das auszugebende Diagramm mit der mindestens einen Alarmierungsgrenze zu bestimmen und über das Ausgabesignal bereitzustellen. Durch das vorzugsweise visuelle Ausgeben der Alarmierungsgrenze kann ein Nutzer schnell erfassen, ob die Messwerte des Messwertverlaufs oder zumindest die Verlaufskurvenaufspreizung in die Nähe der Alarmierungsgrenze gelangt. Insbesondere kann eingeschätzt werden, ob bei einem Überschreiten der Alarmierungsgrenze eine besonders große Verlaufskurvenaufspreizung, also eine besonders geringe Signalgüte vorlag.

Gemäß einem zweiten Aspekt der Erfindung wird zur Lösung der oben genannten Aufgabe ein Ausgabesystem zum Ausgeben eines zeitlichen Messwertverlaufs eines medizinischen Messwerts vorgeschlagen. Das Ausgabesystem umfasst dabei eine Ausgabeeinrichtung gemäß mindestens einer der vorhergehenden Ausführungsformen und eine Datenerfassungseinrichtung. Die Datenerfassungseinrichtung ist dabei ausgebildet, die Sensordaten über ein empfangenes Sensorsignal zu erfassen, die Signalgüte des Sensorsignals zu bestimmen und den Sensordaten die entsprechenden Signalgütedaten, insbesondere die entsprechenden aktuellen Signalgütedaten, zuzuordnen. Weiterhin ist die Datenerfassungseinrichtung ausgebildet, die Sensordaten und die zugeordneten Signalgütedaten an die Ausgabeeinrichtung auszugeben. Die Ausgabe kann dabei direkt an die Ausgabeeinrichtung, insbesondere in Form des Empfangssignals, erfolgen oder indirekt über ein weiteres Gerät. Das weitere Gerät kann beispielsweise ein Gerät sein, welches ein Signal von einem ersten Signalprotokoll in ein zweites Signalprotokoll übersetzt. Hierdurch kann eine Kompatibilität zwischen verschiedenen Geräten und/oder zwischen Geräten von verschiedenen Herstellern besonders vorteilhaften möglich sein.

Vorzugsweise basiert die bestimmte Signalgüte auf dem Signal-Rausch-Verhältnis des empfangenen Sensorsignals. Alternativ oder ergänzend kann die Bestimmung der Signalgüte beispielsweise über den sogenannten SINAD-Wert (signal-to-interference ratio including noise and distortion) erfolgen. Alternativ oder ergänzend kann ein Dynamikbereich der aufgenommenen Daten, beispielsweise unter Berücksichtigung einer Körnung der Messung, wie etwa einer Pixelbreite, einer Sensorauflösung und dergleichen, berücksichtigt werden.

In einer besonders vorteilhaften Ausführungsform des Ausgabesystems ist die Datenerfassungseinrichtung ausgebildet, eine Korrelation zwischen den Sensordaten und den zugeordneten Signalgütedaten über die Zeit zu bestimmen und an die Ausgabeeinrichtung auszugeben, wobei die Ausgabeeinrichtung weiter ausgebildet ist, die Aufspreizung des Messwertverlaufs basierend auf der Korrelation zu bestimmen. Ist die Signalgüte beispielsweise völlig unabhängig von den Sensordaten immer auf einem ähnlichen Niveau, kann vorteilhaft ein zeitlicher Abstand zwischen Zeitpunkten, in denen die Verlaufskurvenaufspreizung bestimmt wird vergrößert werden, da keine Änderung der Verlaufskurvenaufspreizung zu erwarten ist. Alternativ oder ergänzend kann ein Typ von Sensordaten eine starke Korrelation mit der zugeordneten Signalgüte aufweisen, sodass für einen konkreten Typ von Sensordaten stets eine ähnliche Signalgüte vorliegt. Der Typ der Sensordaten kann dabei ein Daten-Typ, ein Messwert-Typ, ein Geräte-Typ für die Bestimmung der Messwerte oder dergleichen sein.

In einer bevorzugten Ausführungsform ist das Ausgabesystem ausgebildet, eine Kommunikationsverbindung mit einem externen Alarmierungssystem bereitzustellen und ein Warnsignal bereitzustellen, falls eine aktuell bestimmte Signalgüte des Sensorsignals unterhalb eines vorbestimmten Grenzwerts liegt. In dieser Ausführungsform wird ein Nutzer des Alarmierungssystem sowie ein Nutzer des Ausgabesystems darauf hingewiesen, dass die aktuell bestimmte Signalgüte unterhalb des vorbestimmten Grenzwerts liegt. Hierdurch kann eine Reaktion des Nutzers, wie beispielsweise eine Reparatur der entsprechenden Verbindung oder ein erneuter Verbindungsaufbau initiiert werden. In einer Variante dieser Ausführungsform ist das Ausgabesystem weiterhin dazu ausgeführt, ein Warnsignal bereitzustellen falls ein Messwert innerhalb des auszugebenden Messwertverlaufs einen vorbestimmten Schwellenwert passiert. Das Bereitstellen des Warnsignals erfolgt vorzugsweise über die Kommunikationsverbindung durch das externen Alarmierungssystem. Alternativ und/oder ergänzend kann das Warnsignal zusätzlich oder ausschließlich durch das Ausgabesystem optische und/oder akustisch an einen Nutzer des Ausgabesystems ausgegeben werden.

In einer vorteilhaften Ausführungsform weist das Ausgabesystem weiterhin eine Benutzerschnittstelle auf, die ausgebildet ist, eine Nutzereingabe zu empfangen, wobei die Nutzereingabe einen Betriebsmodus des Ausgabesystem, eine Auswahl einer graphischen Darstellung für das auszugebende Diagramm, insbesondere der darzustellenden Aufspreizung des zeitlichen Messwertverlaufs, aus einer hinterlegten Mehrzahl von graphischen Darstellungen und/oder eine Auswahl einer Bestimmungsvorschrift für die Bestimmung der Signalgüte aus einer hinterlegten Mehrzahl von Bestimmungsvorschriften betrifft. Der auszuwählende Betriebsmodus des Ausgabesystems kann beispielsweise ein Aktivieren oder ein Deaktivieren des Ausgabesystems umfassen. In dieser Ausführungsform können die graphischen Darstellungen an die Vorlieben des Nutzers des Ausgabesystems angepasst werden.

Gemäß einem dritten Aspekt der Erfindung wird zur Lösung der oben genannten Aufgabe ein Verfahren zum Ausgeben eines zeitlichen Messwertverlaufs eines medizinischen Messwerts vorgeschlagen. Das erfindungsgemäße Verfahren weist die folgenden Schritte auf:
- Empfangen und Bereitstellen von Sensordaten und Signalgütedaten, wobei die Sensordaten den zeitlichen Messwertverlauf des medizinischen Messwerts indizieren, und wobei die Signalgütedaten einen dem zeitlichen Messwertverlauf zugeordneten zeitlichen Signalgüteverlauf indizieren;
- Bestimmen eines auszugebenden Diagramms mit einer Messwertverlaufskurve basierend auf den Sensordaten und den Signalgütedaten und Bereitstellen eines entsprechenden Ausgabesignals, wobei die Messwertverlaufskurve den zeitlichen Messwertverlauf und eine Verlaufskurvenaufspreizung des Messwertverlaufs anzeigt, und wobei die Messwertverlaufskurve durch ihre Lage und Struktur innerhalb des auszugebenden Diagramms den zeitlichen Messwertverlauf des medizinischen Messwerts indiziert, und wobei die jeweils einem Zeitpunkt der Messwertverlaufskurve zugeordnete Verlaufskurvenaufspreizung ein Maß für die zu diesem Zeitpunkt der Messwertverlaufskurve entsprechend der Signalgütedaten vorliegende Signalgüte ist; und
- Empfangen des Ausgabesignals und graphisches Ausgeben des auszugebenden Diagramms mit der Messwertverlaufskurve, wobei die Verlaufskurvenaufspreizung als entsprechende Aufspreizung des zeitlichen Messwertverlaufs dargestellt wird.

Die Schritte des erfindungsgemäßen Verfahrens werden in der vorgestellten Reihenfolge ausgeführt. So werden die Sensordaten und die Signalgütedaten erst empfangen, ehe basierend auf diesen das Ausgabesignal mit der Messwertverlaufskurve bestimmt und daraufhin grafisch ausgegeben wird.

Vorzugsweise werden die Verfahrensschritte zumindest nahezu in Echtzeit ausgeführt, sodass zwischen dem Empfang der Daten und der graphischen Ausgabe des auszugebenden Diagramms weniger als 5 Sekunden, vorzugsweise weniger als 2 Sekunden, besonders bevorzugt weniger als 1 Sekunde liegt. Hierdurch kann ein Anwender des erfindungsgemäßen Verfahrens besonders schnell den aktuellen Zustand des Patienten und die aktuelle Signalgüte über die graphische Ausgabe erkennen.

Vorzugsweise werden die Schritte des erfindungsgemäßen Verfahrens von einem einzigen Gerät ausgeführt. Alternativ kann zumindest einer der Verfahrensschritte auf einem beabstandeten Gerät ausgeführt werden.

Gemäß einem vierten Aspekt der Erfindung wird zur Lösung der oben genannten Aufgabe ein Computerprogramm mit einem Programmcode zur Durchführung eines Verfahrens gemäß dem dritten Aspekt der Erfindung vorgeschlagen, wenn der Programmcode auf einem Computer, einem Prozessor oder einer programmierbaren Hardwarekomponente ausgeführt wird. Vorzugsweise werden mehrere Schritte des erfindungsgemäßen Verfahrens durch einen gemeinsamen Computer, einen gemeinsamen Prozessor oder eine gemeinsame programmierbare Hardwarekomponente ausgeführt. Vorzugsweise sind die einzelnen Schritte dabei zumindest auf Software-Ebene voneinander durch entsprechende Softwareblöcke getrennt. Besonders bevorzugt werden alle Schritte des erfindungsgemäßen Verfahrens auf einem gemeinsamen Computer, einem gemeinsamen Prozessor oder einer gemeinsamen programmierbaren Hardwarekomponente ausgeführt.

Die Erfindung soll nun anhand von in den Figuren schematisch dargestellten, vorteilhaften Ausführungsbeispielen näher erläutert werden. Von diesen zeigen im Einzelnen:
- Fig. 1: eine schematische Darstellung eines ersten Ausführungsbeispiels einer Ausgabeeinrichtung gemäß einem ersten Aspekt der Erfindung;
- Figs. 2, 3, 4: eine schematische Darstellung einer Messwertverlaufskurve einer Ausgabeeinrichtung gemäß dem ersten Aspekt der Erfindung, wobei eine diskontinuierliche Aufspreizung (Fig. 2), eine kontinuierliche Aufspreizung (Fig. 3) und eine diskrete Aufspreizung (Fig. 4) dargestellt sind;
- Fig. 5: eine schematische Darstellung eines zweiten Ausführungsbeispiels der Ausgabeeinrichtung gemäß dem ersten Aspekt der Erfindung;
- Fig. 6: eine schematische Darstellung eines ersten Ausführungsbeispiels eines Ausgabesystems gemäß einem zweiten Aspekt der Erfindung;
- Fig. 7: eine schematische Darstellung eines zweiten Ausführungsbeispiels des Ausgabesystems gemäß dem zweiten Aspekt der Erfindung;
- Fig. 8: ein Flussdiagramm eines Ausführungsbeispiels eines Verfahrens gemäß einem dritten Aspekt der Erfindung.

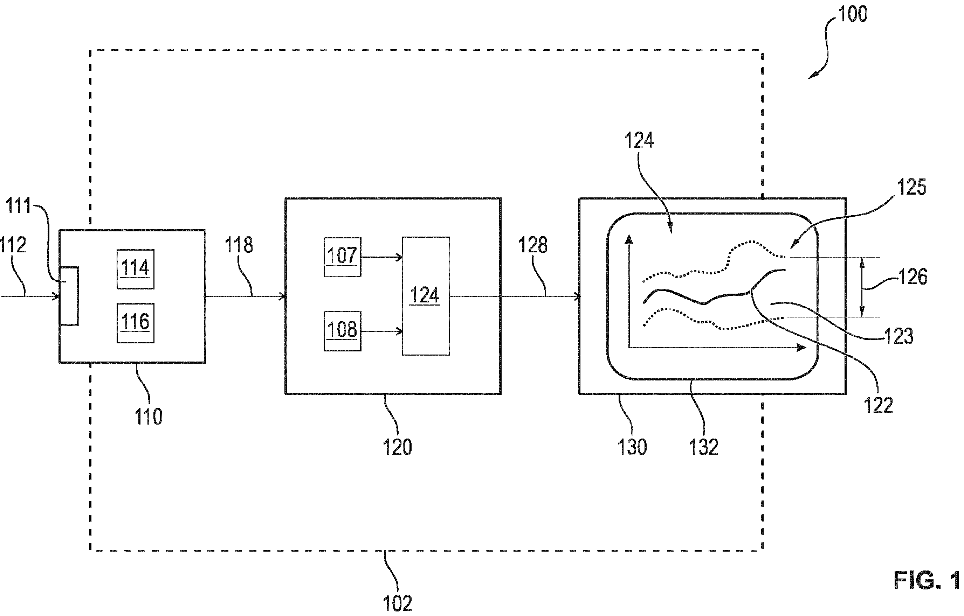

Fig. 1 zeigt eine schematische Darstellung eines ersten Ausführungsbeispiels einer Ausgabeeinrichtung 100 gemäß einem ersten Aspekt der Erfindung.

Die Ausgabeeinrichtung 100 ist zum Ausgeben eines zeitlichen Messwertverlaufs 122 eines medizinischen Messwerts 107 ausgebildet. Hierfür weist die Ausgabeeinrichtung 100 eine Empfangseinheit 110, eine Ausgabesteuerungseinheit 120 und eine Displayeinheit 130 auf.

Die Empfangseinheit 110 ist ausgebildet, Sensordaten 114 und Signalgütedaten 116 über mindestens ein Empfangssignal 112 zu empfangen und zur Weiterverarbeitung durch die Ausgabeeinrichtung 100 bereitzustellen. Das Bereitstellen erfolgt in dem dargestellten Ausführungsbeispiel über ein Verarbeitungssignal 118. Die Sensordaten 114 indizieren den zeitlichen Messwertverlauf 122 des medizinischen Messwerts 107, und die Signalgütedaten 116 indizieren einen dem zeitlichen Messwertverlauf 122 zugeordneten zeitlichen Signalgüteverlauf. Vorliegend werden die Sensordaten 114 und die Signalgütedaten 116 von einem externen nicht dargestellten Gerät bereitgestellt, mit dem die Empfangseinheit 110 kabelbasiert kommuniziert. Hierfür verfügt die Empfangseinheit 110 über eine Empfangsschnittstelle 111 zum Empfangen des Empfangssignals 112. Die Sensordaten 114 und die Signalgütedaten 116 werden vorliegend durch ein einziges Empfangssignal 112 bereitgestellt, welches die entsprechenden Daten transportiert. Alternativ oder ergänzend können mehrere Empfangssignale zum Empfangen der Daten oder anderer Informationen vorgesehen sein.

Die Ausgabesteuerungseinheit 120 ist ausgebildet, basierend auf den Sensordaten 114 und den Signalgütedaten 116 ein auszugebendes Diagramm 124 mit einer Messwertverlaufskurve 125 zu bestimmen und über ein Ausgabesignal 128 bereitzustellen. Dabei zeigt die Messwertverlaufskurve 125 den zeitlichen Messwertverlauf 122 und eine Verlaufskurvenaufspreizung 123 des Messwertverlaufs 122 an. Die Messwertverlaufskurve 125 indiziert durch ihre Lage und Struktur innerhalb des auszugebenden Diagramms 124 den zeitlichen Messwertverlauf 122 des medizinischen Messwerts 107. Dies kann beispielsweise, wie aus dem dargestellt Ausführungsbeispiel ersichtlich, über eine Lage innerhalb eines Koordinatensystems erfolgen. In anderen dargestellten Ausführungsbeispielen erfolgt dies über Markierungen, insbesondere vereinzelte Markierungen, die eine Größenordnung des entsprechenden Messwerts und/oder des entsprechenden Zeitpunktes anzeigen. Wobei die jeweils einem Zeitpunkt der Messwertverlaufskurve 125 zugeordnete Verlaufskurvenaufspreizung 123 ein Maß für die zu diesem Zeitpunkt der Messwertverlaufskurve 125 entsprechend der Signalgütedaten 116 vorliegende Signalgüte 108 ist.

Wie in Fig. 1 schematisch dargestellt, werden aus den Sensordaten 114 und den Signalgütedaten 116 jeweils Werte für den medizinischen Messwert 107 und die Signalgüte 108 bestimmt und zu dem auszugebenden Diagramms 124 kombiniert.

Die Displayeinheit 130 ist ausgebildet, das entsprechende Ausgabesignal 128 zu empfangen und auf einem Display 132 der Displayeinheit 130 das auszugebende Diagramm 124 mit der Messwertverlaufskurve 125 und der entsprechenden Verlaufskurvenaufspreizung 123 graphisch auszugeben. Dabei ist die Verlaufskurvenaufspreizung 123 als entsprechende Aufspreizung 126 des zeitlichen Messwertverlaufs 122 dargestellt.

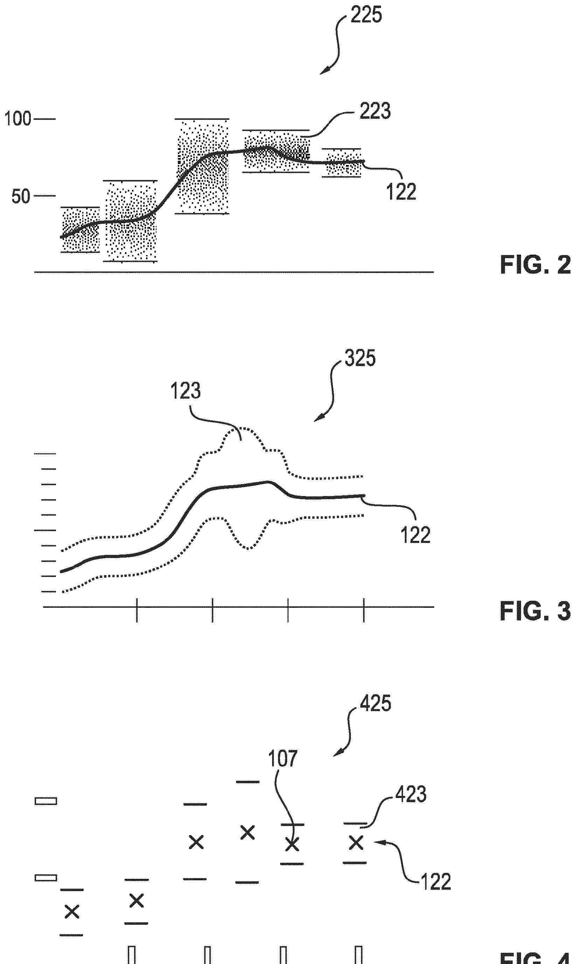

Dieser Aufspreizung 126 des zeitlichen Messwertverlaufs 122 kann erfindungsgemäß auf verschiedene Weisen erfolgen. In dem dargestellten Ausführungsbeispiel ist eine asymmetrische Aufspreizung ausgehend von dem Messwertverlauf 122 dargestellt. Im Rahmen der Figuren 2 bis 4 sind symmetrische Aufspreizungen des entsprechenden Messwertverlaufs dargestellt.

Der Messwertverlauf 122 ist in dem dargestellten Ausführungsbeispiel durch eine durchgezogene kontrastreiche Linie kontrastreicher dargestellt als ein über eine gepunktete Darstellung angedeuteter Randbereich der dargestellten Verlaufskurvenaufspreizung 123. Die Aufspreizung 126 ist jeweils eine kontinuierlich verlaufende Aufspreizung um den Messwertverlauf 122 und mithin um die den Messwertverlauf 122 indizierenden Bereiche der Messwertverlaufskurve 125, nämlich vorliegend die durchgezogene Linie, dargestellt. Das Diagramm 124 ist in dem dargestellten Ausführungsbeispiel über eine X-Achse und eine Y-Achse als Diagramm erkennbar. Die X-Achse beschreibt hierbei ein Zeitintervall, wohingegen die Y-Achse von dem untersuchten medizinischen Messwert 107 abhängig ist.

Die Aufspreizung ist umgekehrt proportional zu der zu diesem entsprechenden Zeitpunkt der Aufspreizung entsprechend der Signalgütedaten 116 vorliegenden Signalgüte 108.

In dem dargestellten Ausführungsbeispiel handelt es sich bei der Signalgüte 108 um Werte, die auf dem Signal-Rausch-Verhältnis eines vorher empfangenen Sensorsignals basieren. Dieses Verhältnis wurde von einem nicht dargestellten externen Gerät bestimmt und über das Empfangssignal 112 an die erfindungsgemäße Ausgabeeinrichtung 100 ausgegeben.

Die Einheiten der erfindungsgemäßen Ausgabeeinrichtung 100 können, wie in dem dargestellten Ausführungsbeispiel gezeigt, in einem gemeinsamen Gehäuse 102 angeordnet sein. Alternativ oder ergänzend können einzelne Einheiten der erfindungsgemäßen Ausgabeeinrichtung beabstandet voneinander, insbesondere in separaten Gehäusen, angeordnet sein. Für beanstandete Einheiten ist eine kabellose Kommunikation besonders vorteilhaft. Eine derartige kabellose Kommunikation kann über ein Netzwerk, über Bluetooth, über BLE, über ZigBee oder dergleichen erfolgen.

In dem dargestellten Ausführungsbeispiel sind die Einheiten zumindest auf Softwareebene voneinander getrennt und werden von einem gemeinsamen nicht dargestellten Prozessor ausgeführt.

Die Figuren 2, 3 und 4 zeigen eine schematische Darstellung einer jeweiligen Messwertverlaufskurve 225, 325, 425 einer Ausgabeeinrichtung gemäß dem ersten Aspekt der Erfindung, wobei eine diskontinuierliche Aufspreizung (Fig. 2), eine kontinuierliche Aufspreizung (Fig. 3) und eine diskrete Aufspreizung (Fig. 4) dargestellt sind.

Die Messwertverläufe 122 sind für die dargestellten Messwertverlaufskurven 225, 325 und 425 identisch, sodass sich die dargestellten schematische Darstellungen nur in der Art der Darstellung des Diagramms, des Messwertverlaufs und in der Größe und der Art der Darstellung der Aufspreizung unterscheiden.

Fig. 2 zeigt eine Y-Achse mit nur zwei verschiedenen Werten, um die aktuelle Größenordnung des vorliegenden Messwerts einzuschätzen. Die X-Achse stellt einen Zeitbereich dar. Dieser Zeitbereich ist ohne Markierungen oder dergleichen dargestellt.

Der Messwertverlauf 122 ist als durchgezogene Linie dargestellt. Die Verlaufskurvenaufspreizung 223 ist symmetrisch um einen durch die Sensordaten indizierten Messwert als Mittelpunkt der Aufspreizung dargestellt. Dabei orientiert sich die Größe der Aufspreizung an einem einzigen Messwert innerhalb eines vorbestimmten Zeitintervalls. Die sich daraus ergebende Aufspreizung wird für das gesamte Zeitintervall dargestellt. Das sich durch die Aufspreizung ergebende Intervall für die Größe des Messwerts ist im Bereich des Messwertverlaufs 122 kontrastreich dargestellt und wird zu den Rändern der Aufspreizung hin kontrastärmer.

Fig. 3 zeigt eine Y-Achse ohne explizit ausgeschriebene Werte aber mit Strichen zur Darstellung der Größenordnung des aktuell vorliegenden Messwerts. Die X-Achse stellt einen Zeitbereich dar. Dieser Zeitbereich wird durch einzelne Markierungen in vorbestimmten Zeitintervallen optisch erkennbar.

Die Darstellung der Verlaufskurvenaufspreizung 123 erfolgt wie in Fig. 1 dargestellt über eine kontrastarme Darstellung eines Randbereichs dieser Aufspreizung. Dabei ist sowohl der Messwertverlauf 122 als auch der Verlauf der Verlaufskurvenaufspreizung 123 kontinuierlich dargestellt, sodass jedem einzelnen dargestellten Messwert eine diesem Messwert zuzuordnende Signalgüte erkennbar ist.

Fig. 4 zeigt sowohl entlang der Y-Achse als auch entlang der X-Achse lediglich vereinzelte Markierungen, die einen entsprechenden Bereich des dargestellten Zeitintervalls und des dargestellten Messwertintervalls optisch erkennbar machen.

Die Darstellung der Verlaufskurvenaufspreizung 423 erfolgt in diskreten Schritten entsprechend der Darstellung des Messwertverlaufs 122, der ebenfalls durch diskret dargestellte Messwerte 107 visualisiert ist. Jedem dargestellten Messwert 107 ist mithin eine Aufspreizung zugeordnet, die die dargestellte Verlaufskurvenaufspreizung 423 ergibt.

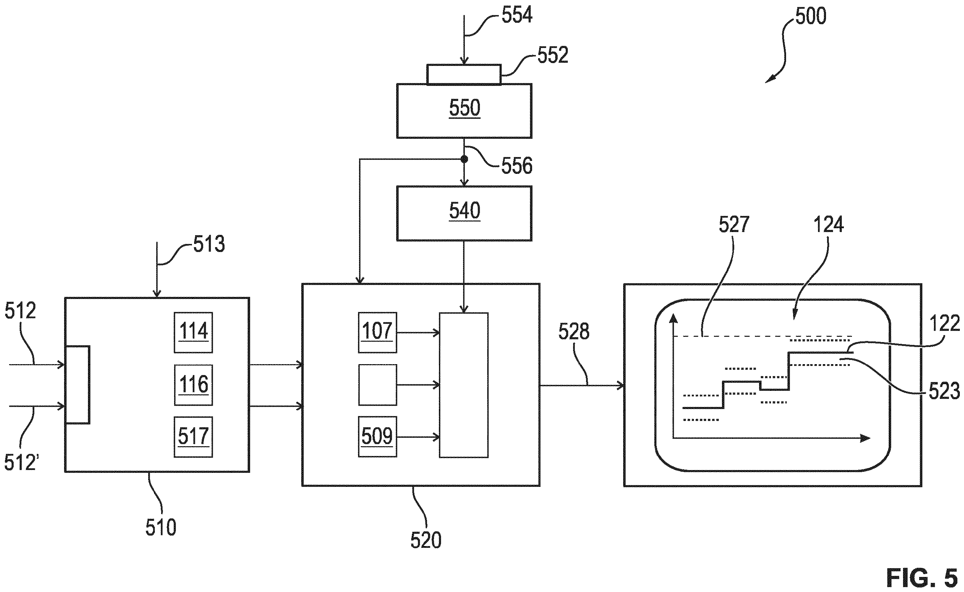

Fig. 5 zeigt eine schematische Darstellung eines zweiten Ausführungsbeispiels der Ausgabeeinrichtung 500 gemäß dem ersten Aspekt der Erfindung.

Die Ausgabeeinrichtung 500 unterscheidet sich dadurch von der in Fig. 1 dargestellten Ausgabeeinrichtung 100, dass die Empfangseinheit 510 dazu ausgebildet ist, zwei Empfangssignale 512, 512' zu empfangen, wobei ein Empfangssignal 512 die Sensordaten 114 indiziert und das weitere Empfangssignal 512' die Signalgütedaten 116 indiziert. In einem alternativen oder ergänzenden Ausführungsbeispiel werden in zeitlichen Abständen verschiedene Empfangssignale durch die erfindungsgemäße Empfangseinheit empfangen, die beispielsweise Messwerte zu verschiedenen Zeitpunkten und/oder durch unterschiedliche externe Geräte ausgewertete Messwerte indizieren.

Zudem ist die Empfangseinheit 510 dazu ausgebildet Alarmierungsdaten 517 über ein Alarmierungssignal 513 zu empfangen. Die Alarmierungsdaten 517 indizierende mindestens eine Alarmierungsgrenze für den medizinischen Messwert 107. Zudem ist die Ausgabesteuerungseinheit 520 dazu ausgebildet, die mindestens eine auszugebende Darstellung einer Alarmierungsgrenze 509 zu bestimmen und über das Ausgabesignal 528 bereitzustellen. Die Alarmierungsgrenze 509 wird als Alarmierungslinie 527 in dem Diagramm 124 dargestellt.

Weiterhin umfasst die Ausgabeeinrichtung 500 ein Speichermodul 540, auf dem eine Mehrzahl von graphischen Darstellungen für das auszugebende Diagramm 124 hinterlegt ist. Das Speichermodul 540 ist dazu ausgebildet, eine aktuell vorbestimmte graphische Darstellung an die Ausgabesteuerungseinheit 520 auszugeben, damit diese die aktuell vorbestimmte graphische Darstellung für die Bestimmung des auszugebenden Diagramms 124 verwendet.

Schließlich umfasst die Ausgabeeinrichtung 500 im Gegensatz zu der Ausgabeeinrichtung 100 eine Eingabeeinheit 550, die dazu ausgebildet ist, über eine Eingabeschnittstelle 552 eine Benutzereingabe 554 zu empfangen und ein entsprechendes Eingabesignal 556 an das Speichermodul 540 und/oder an die Ausgabesteuerungseinheit 520 auszugeben. Das Eingabesignal 552 kann dabei eine Auswahl der als graphische Darstellung zu verwendenden aktuell vorbestimmten graphischen Darstellung indizieren, einen aktuellen Betriebsmodus der Ausgabeeinrichtung 500, wie etwa ein Aktivieren oder Deaktivieren, indizieren und/oder ein Aktivieren oder Deaktivieren der Ausgabe der Alarmierungsgrenze 509 indizieren.

In einem nicht dargestellten Ausführungsbeispiel kann das Eingabesignal einen Empfangsmodus für die erfindungsgemäße Empfangseinheit und/oder einen Ausgabemodus der erfindungsgemäßen Displayeinheit indizieren.

Der Messwertverlauf 122 wird in diesem Ausführungsbeispiel als ein stetiger Verlauf dargestellt, der mit jedem neuen Messwert eine entsprechende Sprungstelle zu diesem Messwert aufweist. Entsprechend weist die Verlaufskurvenaufspreizung 523 ebenfalls eine Sprungstelle für jeden neuen diskreten Messwert auf.

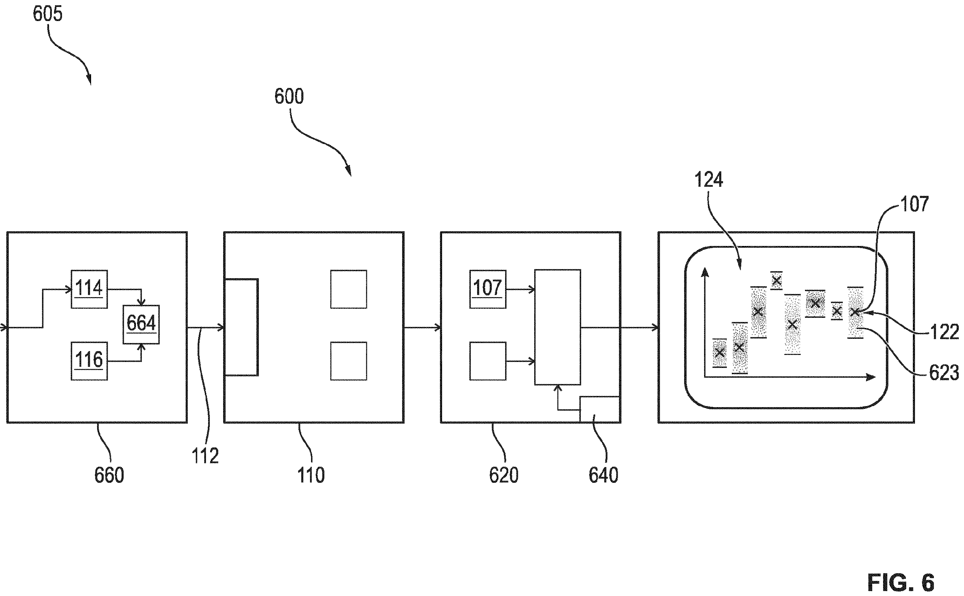

Fig. 6 zeigt eine schematische Darstellung eines ersten Ausführungsbeispiels eines Ausgabesystems 605 gemäß einem zweiten Aspekt der Erfindung.

Das Ausgabesystem 605 ist zum Ausgeben des zeitlichen Messwertverlaufs 122 eines medizinischen Messwerts 107 ausgebildet. Hierfür umfasst es eine Ausgabeeinrichtung 600 entsprechend dem ersten Aspekt der Erfindung und eine Datenerfassungseinrichtung 660.

Die Datenerfassungseinrichtung 660 ist ausgebildet, die Sensordaten 114 über ein empfangenes Sensorsignal 662 zu erfassen, die Signalgüte des Sensorsignals 662 zu bestimmen und den Sensordaten 114 die entsprechenden Signalgütedaten 116, insbesondere die entsprechenden aktuellen Signalgütedaten, zuzuordnen. In dem dargestellten Ausführungsbeispiel werden die Sensordaten 114 mit den entsprechend zugeordneten Signalgütedaten 116 zu einem kombinierten Datensatz 664 zusammengeführt, der dann ausgegeben wird. Die Ausgabe des kombinierten Datensatzes 662 kann an ein Netzwerk, an ein Patientendaten-Management-System oder dergleichen erfolgen. In dem dargestellten Ausführungsbeispiel wird direkt das durch die Empfangseinheit 110 empfangener Empfangssignal 112 durch die Datenerfassungseinrichtung 660 als Ausgabe bereitgestellt.

Das Speichermodul 640 ist in dem dargestellten Ausführungsbeispiel kein separates Modul, sondern ein Teil der Ausgabesteuerungseinheit 620.

Das dargestellte Diagramm 124 zeigt diskrete Messwerte 107, mit einer entsprechenden diskreten Verlaufskurvenaufspreizung 623. Die Verlaufskurvenaufspreizung 623 zeigt einen Verlauf von einem kontrastreichen Zentrum im Bereich der Messwerte 107 zu einem kontrastarmen Randbereich dieser Aufspreizung.

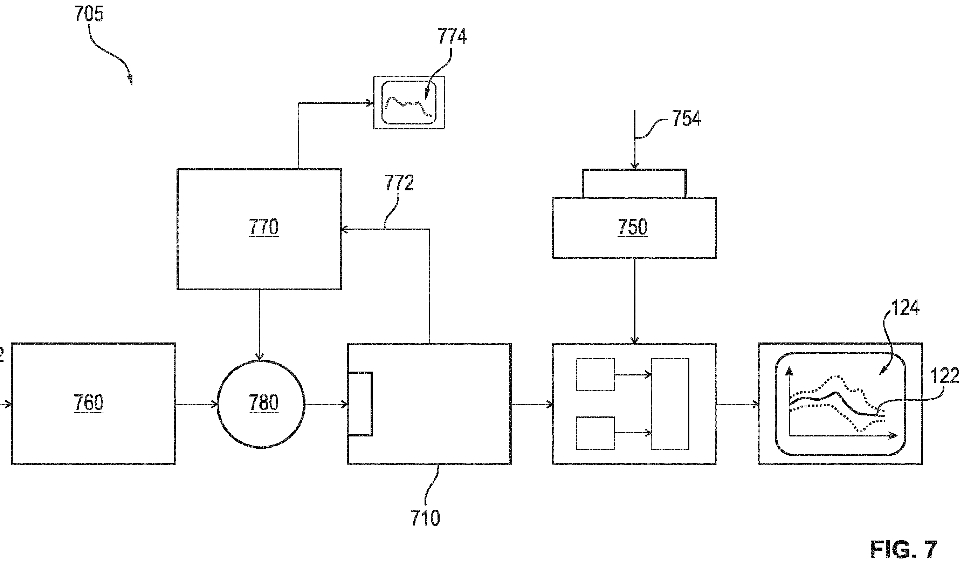

Fig. 7 zeigt eine schematische Darstellung eines zweiten Ausführungsbeispiels des Ausgabesystems 705 gemäß dem zweiten Aspekt der Erfindung.

Das Ausgabesystem 705 unterscheidet sich dadurch von dem in Fig. 6 dargestellten Ausgabesystem 605, dass es eine Kommunikationsverbindung mit einem externen Alarmierungssystem 770 aufweist. Die Kommunikationsverbindung erfolgt hierbei über ein Netzwerk 780, über welches die Datenerfassungseinrichtung 760 mit der Empfangseinheit 710 verbunden ist. Zudem wird die Kommunikationsverbindung durch ein Rückkanal gebildet, über den die Empfangseinheit 710 ein Warnsignal 772 ausgeben kann, falls eine aktuell bestimmte Signalgüte des Sensorsignals 662 unterhalb eines vorbestimmten Grenzwerts liegt. In einem alternativen oder ergänzenden Ausführungsbeispiel ist die Datenerfassungseinrichtung dazu ausgebildet ein solches Warnsignal an ein externes Alarmierungssystem auszugeben. In einem weiteren alternativen oder ergänzenden Ausführungsbeispiel ist die Ausgabesteuerungseinheit dazu ausgebildet, ein solches Warnsignal an ein externes Alarmierungssystem auszugeben.

Das externe Alarmierungssystem 770 umfasst eine optische Ausgabe 774, durch die das Unterschreiten eines vorbestimmten Grenzwerts für die aktuell bestimmte Signalgüte angezeigt wird.

Schließlich umfasst das Ausgabesystem 705 eine Eingabeeinheit 750, über die, wie bereits für die Eingabeeinheit 550 beschrieben, eine Nutzereingabe 554 empfangen und weiterverarbeitet werden kann. Dabei betrifft die Nutzereingabe 554 einen Betriebsmodus des Ausgabesystems 705, eine Auswahl einer graphischen Darstellung für das auszugebende Diagramm 124, insbesondere der darzustellenden Aufspreizung des zeitlichen Messwertverlaufs 122, aus einer hinterlegten Mehrzahl von graphischen Darstellungen und/oder eine Auswahl einer Bestimmungsvorschrift für die Bestimmung der Signalgüte aus einer hinterlegten Mehrzahl von Bestimmungsvorschriften.

Alternativ oder ergänzend kann eine Dauer eines zurückliegenden Zeitbereichs, der zusammen mit dem auszugebenden Diagramm darzustellen ist, über die Nutzereingabe eingestellt werden.

In dem dargestellten Ausführungsbeispiel wird die bestimmte Signalgüte vorzugsweise basierend auf dem Signal-Rausch-Verhältnis des empfangenen Sensorsignals 662 bestimmt.

Das Diagramm 124 ist ein Diagramm mit einer asymmetrischen Verlaufskurvenaufspreizung um den Messwertverlauf 122 herum. Die grafische Darstellung erfolgt dabei entsprechend dem in Fig. 1 dargestellten Ausführungsbeispiel.

In einem nicht dargestellten Ausführungsbeispiel ist die Datenerfassungseinrichtung zudem ausgebildet, eine Korrelation zwischen den Sensordaten und den zugeordneten Signalgütedaten über die Zeit zu bestimmen und auszugeben, wobei die Aufspreizung des Messwertverlaufs vorzugsweise basierend auf dieser Korrelation bestimmt wird.

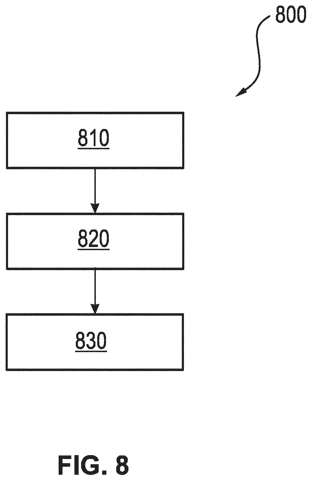

Fig. 8 zeigt ein Flussdiagramm eines Ausführungsbeispiels eines Verfahrens 800 gemäß einem dritten Aspekt der Erfindung.

Das erfindungsgemäße Verfahren 800 ist zum Ausgeben eines zeitlichen Messwertverlaufs eines medizinischen Messwerts ausgebildet. Das Verfahren 800 weist dabei die im Folgenden erläuterten Schritte auf.

Ein erster Schritt 810 umfasst ein Empfangen und ein Bereitstellen von Sensordaten und Signalgütedaten, wobei die Sensordaten den zeitlichen Messwertverlauf des medizinischen Messwerts indizieren, und wobei die Signalgütedaten einen dem zeitlichen Messwertverlauf zugeordneten zeitlichen Signalgüteverlauf indizieren.

Ein darauffolgender Schritt 820 umfasst ein Bestimmen eines auszugebenden Diagramms mit einer Messwertverlaufskurve basierend auf den Sensordaten und den Signalgütedaten und ein Bereitstellen eines entsprechenden Ausgabesignals, wobei die Messwertverlaufskurve den zeitlichen Messwertverlauf und eine Verlaufskurvenaufspreizung des Messwertverlaufs anzeigt, und wobei die Messwertverlaufskurve durch ihre Lage und Struktur innerhalb des auszugebenden Diagramms den zeitlichen Messwertverlauf des medizinischen Messwerts indiziert, und wobei die jeweils einem Zeitpunkt der Messwertverlaufskurve zugeordnete Verlaufskurvenaufspreizung ein Maß für die zu diesem Zeitpunkt der Messwertverlaufskurve entsprechend der Signalgütedaten vorliegende Signalgüte ist.

Schließlich umfasst ein abschließender Schritt 830 ein Empfangen des Ausgabesignals und ein graphisches Ausgeben des auszugebenden Diagramms mit der Messwertverlaufskurve, wobei die Verlaufskurvenaufspreizung als entsprechende Aufspreizung des zeitlichen Messwertverlaufs dargestellt wird.

Die Schritte 810, 820 und 830 werden in der dargestellten Reihenfolge ausgeführt. So wird stets erst das Empfangssignal empfangen, um die Ausgabe entsprechend zu steuern und schließlich im abschließenden Schritt 830 auszugeben.

Die Ausgabe entsprechend dem Schritt 830 kann dabei zeitgleich mit einem erneuten Empfangen von Sensordaten und Signalgütedaten entsprechend dem Schritt 810 erfolgen. So wird vorzugsweise das erfindungsgemäße Verfahren in kurz hintereinander ausgeführten Zeitschritten wiederholt, um stets eine aktuelle Messwertverlaufskurve darzustellen.

Vorzugsweise vergehen zwischen dem Empfang des Empfangssignals in Schritt 810 und dem Ausgeben des Ausgabesignals in Schritt 830 weniger als 10 Sekunden, vorzugsweise weniger als 5 Sekunden, besonders bevorzugt weniger als 2 Sekunden.

Wie in dem dargestellten Ausführungsbeispielen beschrieben, kann das erfindungsgemäße Verfahren für einen darzustellenden kontinuierlichen Messwertverlauf durchgeführt werden, was vorzugsweise ein besonders schnelles Ausführen des erfindungsgemäßen Verfahrens 800 erfordert. Alternativ oder ergänzend kann das erfindungsgemäß Verfahren für eine diskrete Darstellung von Messwerten, die ein Messen in diskreten Zeitschritten erfordert, durchgeführt werden. Hierbei wird das Verfahren vorzugsweise in kürzeren Zeitschritten hintereinander ausgeführt, als das Zeitintervall zwischen zwei dargestellten Messwerten.

Die Verfahrensschritte können von einem einzigen Gerät, insbesondere von einem einzigen Prozessor ausgeführt werden. Alternativ kann zumindest einer der Verfahrensschritte an einem Ort ausgeführt werden, der beabstandet ist von einem Ort an dem ein anderer dieser Verfahrensschritte ausgeführt wird. Insbesondere kann das erfindungsgemäße Verfahren von mehreren Prozessoren ausgeführt werden.

### Bezugszeichenliste

- 100, 500, 600: Ausgabeeinrichtung
- 102: Gehäuse
- 107: medizinischer Messwert
- 108: Signalgüte
- 110, 510, 710: Empfangseinheit
- 111: Empfangsschnittstelle
- 112, 512, 512`: Empfangssignal
- 114: Sensordaten
- 116: Signalgütedaten
- 118: Verarbeitungssignal
- 120, 520, 620: Ausgabesteuerungseinheit
- 122: Messwertverlauf
- 123, 223, 423, 523, 623: Verlaufskurvenaufspreizung
- 124: auszugebendes Diagramm
- 125, 225, 325, 425: Messwertverlaufskurve
- 126: Aufspreizung
- 128, 528: Ausgabesignal
- 130: Displayeinheit
- 132: Display
- 509: Alarmierungsgrenze
- 513: Alarmierungssignal
- 517: Alarmierungsdaten
- 527: Alarmierungslinie
- 540, 640: Speichermodul
- 550, 750: Eingabeeinheit
- 552: Eingabeschnittstelle
- 554, 754: Benutzereingabe
- 556: Eingabesignal
- 605, 705: Ausgabesystem
- 660, 760: Datenerfassungseinrichtung
- 662: Sensorsignal
- 664: kombinierter Datensatz
- 770: externes Alarmierungssystem
- 772: Warnsignal
- 774: optische Ausgabe
- 780: Netzwerk
- 800: Verfahren
- 810, 820, 830: Verfahrensschritte

## Patentansprüche

1. Ausgabeeinrichtung (100) zum Ausgeben eines zeitlichen Messwertverlaufs (122) eines medizinischen Messwerts (107), mit
- einer Empfangseinheit (110), die ausgebildet ist, Sensordaten (114) und Signalgütedaten (116) über mindestens ein Empfangssignal (112) zu empfangen und zur Weiterverarbeitung durch die Ausgabeeinrichtung (100) bereitzustellen, wobei die Sensordaten (114) den zeitlichen Messwertverlauf (122) des medizinischen Messwerts (107) indizieren, und wobei die Signalgütedaten (116) einen dem zeitlichen Messwertverlauf (122) zugeordneten zeitlichen Signalgüteverlauf indizieren;
- einer Ausgabesteuerungseinheit (120), die ausgebildet ist, basierend auf den Sensordaten (114) und den Signalgütedaten (116) ein auszugebendes Diagramm (124) mit einer Messwertverlaufskurve (125) zu bestimmen und über ein Ausgabesignal (128) bereitzustellen, wobei die Messwertverlaufskurve (125) den zeitlichen Messwertverlauf (122) und eine Verlaufskurvenaufspreizung (123) des Messwertverlaufs (122) anzeigt, und wobei die Messwertverlaufskurve (125) durch ihre Lage und Struktur innerhalb des auszugebenden Diagramms (124) den zeitlichen Messwertverlauf (122) des medizinischen Messwerts (107) indiziert, und wobei die jeweils einem Zeitpunkt der Messwertverlaufskurve (125) zugeordnete Verlaufskurvenaufspreizung (123) ein Maß für die zu diesem Zeitpunkt der Messwertverlaufskurve (125) entsprechend der Signalgütedaten (116) vorliegende Signalgüte ist; und
- einer Displayeinheit (130), die ausgebildet ist, das Ausgabesignal (128) zu empfangen und auf einem Display (132) der Displayeinheit (130) das auszugebende Diagramm (124) mit der Messwertverlaufskurve (125) und der entsprechenden Verlaufskurvenaufspreizung (123) graphisch auszugeben, wobei die Verlaufskurvenaufspreizung (123) als entsprechende Aufspreizung (126) des zeitlichen Messwertverlaufs (122) dargestellt ist.

2. Ausgabeeinrichtung (100) gemäß Anspruch 1, wobei die Aufspreizung (126) eine symmetrische Aufspreizung des Messwertverlaufs (122) um einen durch die Sensordaten (114) indizierten Messwert (107) als Mittelpunkt der Aufspreizung (126) ist.

3. Ausgabeeinrichtung (100) gemäß Anspruch 1 oder 2, wobei den Messwertverlauf (122) indizierende Bereiche der Messwertverlaufskurve (125) kontrastreicher dargestellt sind als die entsprechende Aufspreizung (126).

4. Ausgabeeinrichtung (100) gemäß mindestens einem der vorhergehenden Ansprüche, wobei die Aufspreizung (126) als eine kontinuierlich verlaufende Aufspreizung um die den Messwertverlauf (122) indizierenden Bereiche der Messwertverlaufskurve (125) dargestellt ist.

5. Ausgabeeinrichtung (100) gemäß mindestens einem der vorhergehenden Ansprüche, wobei die Aufspreizung (126) umgekehrt proportional zu der zu diesem Zeitpunkt entsprechend der Signalgütedaten (116) vorliegenden Signalgüte ist.

6. Ausgabeeinrichtung (500) gemäß mindestens einem der vorhergehenden Ansprüche, wobei die Ausgabesteuerungseinheit (520) weiter ausgebildet ist, auf ein Speichermodul (540) mit einer hinterlegten Mehrzahl von graphischen Darstellungen für das auszugebende Diagramm (124) zuzugreifen.

7. Ausgabeeinrichtung (500) gemäß mindestens einem der vorhergehenden Ansprüche, wobei die Empfangseinheit (510) weiter ausgebildet ist, Alarmierungsdaten (517) zu empfangen, wobei die Alarmierungsdaten (517) mindestens eine Alarmierungsgrenze (509) für den medizinischen Messwert (107) indizieren, und wobei die Ausgabesteuerungseinheit (520) weiter ausgebildet ist, das auszugebende Diagramm (124) mit der mindestens einen Alarmierungsgrenze (509) zu bestimmen und über das Ausgabesignal (528) bereitzustellen.

8. Ausgabesystem (605) zum Ausgeben eines zeitlichen Messwertverlaufs (122) eines medizinischen Messwerts (107), mit
- einer Ausgabeeinrichtung (100) gemäß mindestens einem der vorhergehenden Ansprüche, und
- einer Datenerfassungseinrichtung (660), die ausgebildet ist, die Sensordaten (114) über ein empfangenes Sensorsignal (662) zu erfassen, die Signalgüte des Sensorsignals (662) zu bestimmen und den Sensordaten (114) die entsprechenden Signalgütedaten (116), insbesondere die entsprechenden aktuellen Signalgütedaten, zuzuordnen, weiterhin ist die Datenerfassungseinrichtung (660) ausgebildet, die Sensordaten (114) und die zugeordneten Signalgütedaten (116) an die Ausgabeeinrichtung (100) auszugeben.

9. Ausgabesystem (605) gemäß Anspruch 8, wobei die Datenerfassungseinrichtung (660) ausgebildet ist, eine Korrelation zwischen den Sensordaten (114) und den zugeordneten Signalgütedaten (116) über die Zeit zu bestimmen und an die Ausgabeeinrichtung (100) auszugeben, wobei die Ausgabeeinrichtung (100) weiter ausgebildet ist, die Aufspreizung (126) des Messwertverlaufs (112) basierend auf der Korrelation zu bestimmen.

10. Ausgabesystem (705) gemäß Anspruch 8 oder 9, wobei das Ausgabesystem (705) ausgebildet ist, eine Kommunikationsverbindung mit einem externen Alarmierungssystem (770) bereitzustellen und ein Warnsignal (772) bereitzustellen, falls eine aktuell bestimmte Signalgüte des Sensorsignals (662) unterhalb eines vorbestimmten Grenzwerts liegt.

11. Ausgabesystem (705) gemäß mindestens einem der Ansprüche 8 bis 10, wobei das Ausgabesystem (705) weiterhin eine Eingabeschnittstelle (552) aufweist, die ausgebildet ist, eine Nutzereingabe (554) zu empfangen, wobei die Nutzereingabe (554) einen Betriebsmodus des Ausgabesystem (705), eine Auswahl einer graphischen Darstellung für das auszugebende Diagramm (124), insbesondere der darzustellenden Aufspreizung (126) des zeitlichen Messwertverlaufs (122), aus einer hinterlegten Mehrzahl von graphischen Darstellungen und/oder eine Auswahl einer Bestimmungsvorschrift für die Bestimmung der Signalgüte aus einer hinterlegten Mehrzahl von Bestimmungsvorschriften betrifft.

12. Ausgabesystem (705) gemäß mindestens einem der Ansprüche 8 bis 11, wobei die bestimmte Signalgüte auf dem Signal-Rausch-Verhältnis des empfangenen Sensorsignals (662) basiert.

13. Computerimplementiertes Verfahren (800) zum Ausgeben eines zeitlichen Messwertverlaufs (122) eines medizinischen Messwerts (107), aufweisend die Schritte
- Empfangen und Bereitstellen von Sensordaten (114) und Signalgütedaten (116), wobei die Sensordaten (114) den zeitlichen Messwertverlauf (122) des medizinischen Messwerts (107) indizieren, und wobei die Signalgütedaten (116) einen dem zeitlichen Messwertverlauf (122) zugeordneten zeitlichen Signalgüteverlauf indizieren;
- Bestimmen eines auszugebenden Diagramms (124) mit einer Messwertverlaufskurve (125) basierend auf den Sensordaten (114) und den Signalgütedaten (116) und Bereitstellen eines entsprechenden Ausgabesignals (128), wobei die Messwertverlaufskurve (125) den zeitlichen Messwertverlauf (122) und eine Verlaufskurvenaufspreizung (123) des Messwertverlaufs (122) anzeigt, und wobei die Messwertverlaufskurve (125) durch ihre Lage und Struktur innerhalb des auszugebenden Diagramms (124) den zeitlichen Messwertverlauf (122) des medizinischen Messwerts (107) indiziert, und wobei die jeweils einem Zeitpunkt der Messwertverlaufskurve (125) zugeordnete Verlaufskurvenaufspreizung (123) ein Maß für die zu diesem Zeitpunkt der Messwertverlaufskurve (125) entsprechend der Signalgütedaten (116) vorliegende Signalgüte ist; und
- Empfangen des Ausgabesignals (128) und graphisches Ausgeben des auszugebenden Diagramms (124) mit der Messwertverlaufskurve (125), wobei die Verlaufskurvenaufspreizung (123) als entsprechende Aufspreizung (126) des zeitlichen Messwertverlaufs (122) dargestellt wird.

14. Computerprogramm mit einem Programmcode zur Durchführung eines Verfahrens (800) gemäß Anspruch 13, wenn der Programmcode auf einem Computer, einem Prozessor oder einer programmierbaren Hardwarekomponente ausgeführt wird.

## Claims

1. Output device (100) for outputting a temporal measured value profile (122) of a medical measured value (107), comprising
- a receiving unit (110) which is designed to receive sensor data (114) and signal quality data (116) via at least one received signal (112) and to provide them for further processing by the output device (100), wherein the sensor data (114) indicate the temporal measured value profile (122) of the medical measured value (107), and wherein the signal quality data (116) indicate a temporal signal quality profile associated with the temporal measured value profile (122);
- an output control unit (120) which is designed to determine a diagram (124) to be output with a measured value profile curve (125) on the basis of the sensor data (114) and the signal quality data (116) and to provide it via an output signal (128), wherein the measured value profile curve (125) indicates the temporal measured value profile (122) and a profile curve spread (123) of the measured value profile (122), and wherein the measured value profile curve (125) indicates the the temporal measured value profile (122) of the medical measured value (107) by its position and structure within the diagram (124) to be output, and wherein the profile curve spread (123) associated in each case with a time of the measured value profile curve (125) is a measure of the signal quality present at this time of the measured value profile curve (125) in accordance with the signal quality data (116); and
- a display unit (130) which is designed to receive the output signal (128) and to graphically output the diagram (124) to be output with the measurement value profile curve (125) and the corresponding profile curve spread (123) on a display (132) of the display unit (130), wherein the profile curve spread (123) is shown as a corresponding spread (126) of the temporal measured value profile (122).

2. Output device (100) according to claim 1, wherein the spread (126) is a symmetrical spread of the measured value profile (122) around a measured value (107) indexed by the sensor data (114) as the center of the spread (126).

3. Output device (100) according to either claim 1 or claim 2, wherein the regions of the measured value profile (125) indicating the measured value profile curve (122) are shown in greater contrast than the corresponding spread (126).

4. Output device (100) according to at least one of the preceding claims, wherein the spread (126) is shown as a continuously extending spread around the regions of the measured value curve (125) indicating the measured value curve (122).

5. Output device (100) according to at least one of the preceding claims, wherein the spread (126) is inversely proportional to the signal quality present at this time according to the signal quality data (116).

6. Output device (500) according to at least one of the preceding claims, wherein the output control unit (520) is further designed to access a memory module (540) having a stored plurality of graphical representations for the diagram (124) to be output.

7. Output device (500) according to at least one of the preceding claims, wherein the receiving unit (510) is further designed to receive alarm data (517), wherein the alarm data (517) indicate at least one alarm limit (509) for the medical measured value (107), and wherein the output control unit (520) is further designed to determine the diagram (124) to be output with the at least one alarm limit (509) and to provide it via the output signal (528).

8. Output system (605) for outputting a temporal measured value profile (122) of a medical measured value (107), comprising
- an output device (100) according to at least one of the preceding claims, and
- a data acquisition device (660) which is designed to detect the sensor data (114) via a received sensor signal (662), to determine the signal quality of the sensor signal (662), and to assign the corresponding signal quality data (116), in particular the corresponding current signal quality data, to the sensor data (114), furthermore the data acquisition device (660) is designed to output the sensor data (114) and the associated signal quality data (116) to the output device (100).

9. Output system (605) according to claim 8, wherein the data acquisition device (660) is designed to determine a correlation between the sensor data (114) and the associated signal quality data (116) over time and to output them to the output device (100), wherein the output device (100) is further designed to determine the spread (126) of the measured value profile (112) on the basis of the correlation.

10. Output system (705) according to either claim 8 or claim 9, wherein the output system (705) is designed to provide a communication link to an external alarm system (770) and to provide a warning signal (772) if a currently determined signal quality of the sensor signal (662) is below a predetermined limit value.

11. Output system (705) according to at least one of claims 8 to 10, wherein the output system (705) further comprises an input interface (552) which is designed to receive a user input (554), wherein the user input (554) relates to an operating mode of the output system (705), a selection of a graphical representation for the diagram (124) to be output, in particular of the spread (126) of the temporal measured value profile (122) to be represented from a stored plurality of graphical representations and/or a selection of a determination rule for determining the signal quality from a stored plurality of determination rules.

12. Output system (705) according to at least one of claims 8 to 11, wherein the determined signal quality is based on the signal-to-noise ratio of the received sensor signal (662).

13. Computer-implemented method (800) for outputting a temporal measured value profile (122) of a medical measured value (107), comprising the steps of
- receiving and providing sensor data (114) and signal quality data (116), wherein the sensor data (114) indicate the temporal measured value profile (122) of the medical measured value (107), and wherein the signal quality data (116) indicate a temporal signal quality profile associated with the temporal measured value profile (122);
- determining a diagram (124) to be output with a measured value profile curve (125) on the basis of the sensor data (114) and the signal quality data (116) and providing a corresponding output signal (128), wherein the measured value profile curve (125) indicates the temporal measured value profile (122) and a profile curve spread (123) of the measured value curve (122), and wherein the measured value profile curve (125) indicates the temporal measured value profile (122) of the medical measured value (107) by its position and structure within the diagram (124) to be output, and wherein the profile curve spread (123) associated with a time of the measured value profile curve (125) in each case is a measure of the signal quality present at this time of the measured value profile curve (125) corresponding to the signal quality data (116); and
- receiving the output signal (128) and graphically outputting the diagram (124) to be output with the measured value profile curve (125), wherein the profile curve spread (123) is represented as a corresponding spread (126) of the temporal measured value profile (122).

14. Computer program having a program code for carrying out a method (800) according to claim 13 when the program code is executed on a computer, a processor, or a programmable hardware component.

## Revendications

1. Dispositif de sortie (100) destiné à délivrer en sortie une variation temporelle de valeurs mesurées (122) d'une valeur mesurée médicale (107), comportant
- une unité de réception (110) qui est conçue pour recevoir des données de capteur (114) et des données de qualité de signal (116) par le biais d'au moins un signal de réception (112), et pour mettre à disposition ces données pour un traitement ultérieur par le dispositif de sortie (100), dans lequel les données de capteur (114) indexent la variation temporelle de valeurs mesurées (122) de la valeur mesurée médicale (107), et dans lequel les données de qualité de signal (116) indexent une variation temporelle de qualité de signal associée à la variation temporelle de valeurs mesurées (122) ;
- une unité de commande de sortie (120) qui est conçue pour déterminer, sur la base des données de capteur (114) et des données de qualité de signal (116), un diagramme (124) à délivrer en sortie comportant une courbe de variation de valeurs mesurées (125), et pour les mettre à disposition par le biais d'un signal de sortie (128), dans lequel la courbe de variation de valeurs mesurées (125) indique la variation temporelle de valeurs mesurées (122) et un déploiement de courbe de variation (123) de la variation des valeurs mesurées (122), et dans lequel, par son emplacement et sa structure dans le diagramme (124) à délivrer en sortie, la courbe de variation de valeurs mesurées (125) indexe la variation temporelle de valeurs mesurées (122) de la valeur mesurée médicale (107), et dans lequel le déploiement de courbe de variation (123) associé respectivement à un point temporel de la courbe de variation de valeurs mesurées (125) est une mesure de la qualité de signal présente à ce point temporel de la courbe de variation de valeurs mesurées (125) en fonction des données de qualité de signal (116) ; et
- une unité d'affichage (130) qui est conçue pour recevoir le signal de sortie (128) et pour délivrer graphiquement en sortie, sur un écran (132) de l'unité d'affichage (130), le diagramme (124) à délivrer en sortie comportant la courbe de variation de valeurs mesurées (125) et le déploiement de courbe de variation (123) correspondant, dans lequel le déploiement de courbe de variation (123) est représenté comme déploiement (126) correspondant de la variation temporelle de valeurs mesurées (122).

2. Dispositif de sortie (100) selon la revendication 1, dans lequel le déploiement (126) est un déploiement symétrique de la variation de valeurs mesurées (122) autour d'une valeur mesurée (107) indexée par les données de capteur (114) comme point central du déploiement (126).

3. Dispositif de sortie (100) selon la revendication 1 ou 2, dans lequel les zones de la courbe de variation de valeurs mesurées (125), lesquelles zones indexent la variation de valeurs mesurées (122), sont représentées avec un contraste plus élevé que le déploiement (126) correspondant.

4. Dispositif de sortie (100) selon au moins l'une des revendications précédentes, dans lequel le déploiement (126) est représenté comme un déploiement s'étendant de manière continue autour des zones de la courbe de variation de valeurs mesurées (125), lesquelles zones indexent la variation de valeurs mesurées (122).

5. Dispositif de sortie (100) selon au moins l'une des revendications précédentes, dans lequel le déploiement (126) est inversement proportionnel à la qualité de signal présente à ce point temporel en fonction des données de qualité de signal (116).

6. Dispositif de sortie (500) selon au moins l'une des revendications précédentes, dans lequel l'unité de commande de sortie (520) est en outre conçue pour accéder à un module de mémoire (540) comportant une pluralité enregistrée de représentations graphiques du diagramme (124) à délivrer en sortie.

7. Dispositif de sortie (500) selon au moins l'une des revendications précédentes, dans lequel l'unité de réception (510) est en outre conçue pour recevoir des données d'alerte (517), dans lequel les données d'alerte (517) indexent au moins une limite d'alerte (509) pour la valeur mesurée médicale (107), et dans lequel l'unité de commande de sortie (520) est en outre conçue pour déterminer le diagramme (124) à délivrer en sortie comportant l'au moins une limite d'alerte (509) et le mettre à disposition par le biais du signal de sortie (528).

8. Système de sortie (605) destiné à délivrer en sortie une variation temporelle de valeurs mesurées (122) d'une valeur mesurée médicale (107), comportant
- un dispositif de sortie (100) selon au moins l'une des revendications précédentes, et
- un dispositif de collecte de données (660) qui est conçu pour collecter les données de capteur (114) par le biais d'un signal de capteur (662) reçu, pour déterminer la qualité de signal du signal de capteur (662) et pour associer les données de qualité de signal (116) correspondantes, en particulier les données de qualité de signal actuelles correspondantes, aux données de capteur (114), le dispositif de collecte de données (660) étant en coutre conçu pour délivrer en sortie au dispositif de sortie (100) les données de capteur (114) et les données de qualité de signal (116) associées.

9. Système de sortie (605) selon la revendication 8, dans lequel le dispositif de collecte de données (660) est conçu pour déterminer dans le temps une corrélation entre les données de capteur (114) et les données de qualité de signal (116) associées et pour la délivrer en sortie au dispositif de sortie (100), dans lequel le dispositif de sortie (100) est en outre conçu pour déterminer le déploiement (126) de la variation des valeurs mesurées (112) sur la base de la corrélation.

10. Système de sortie (705) selon la revendication 8 ou 9, dans lequel le système de sortie (705) est conçu pour mettre en place une liaison de communication avec un système d'alerte (770) externe, et pour fournir un signal d'avertissement (772) au cas où une qualité de signal déterminée actuellement du signal de capteur (662) se trouve en dessous d'une valeur limite prédéterminée.

11. Système de sortie (705) selon au moins l'une des revendications 8 à 10, dans lequel le système de sortie (705) présente en outre une interface d'entrée (552) qui est conçue pour recevoir une entrée d'utilisateur (554), dans lequel l'entrée d'utilisateur (554) concerne un mode de fonctionnement du système de sortie (705), une sélection d'une représentation graphique pour le diagramme (124) à délivrer en sortie, en particulier du déploiement (126) à représenter de la variation temporelle de valeurs mesurées (122), parmi une pluralité enregistrée de représentations graphiques et/ou une sélection d'une règle de détermination pour la détermination de la qualité de signal, parmi une pluralité enregistrée de règles de détermination.

12. Système de sortie (705) selon au moins l'une des revendications 8 à 11, dans lequel la qualité de signal déterminée est basée sur le rapport signal sur bruit du signal de capteur (662) reçu.

13. Procédé mis en oeuvre par ordinateur (800), destiné à délivrer en sortie une variation temporelle de valeurs mesurées (122) d'une valeur mesurée médicale (107), comprenant les étapes
- réception et mise à disposition de données de capteur (114) et de données de qualité de signal (116), dans lequel les données de capteur (114) indexent la variation temporelle de valeurs mesurées (122) de la valeur mesurée médicale (107), et dans lequel les données de qualité de signal (116) indexent une variation temporelle de qualité de signal associée à la variation temporelle de valeurs mesurées (122) ;
- détermination d'un diagramme (124) à délivrer en sortie comportant une courbe de variation de valeurs mesurées (125) sur la base des données de capteur (114) et des données de qualité de signal (116), et mise à disposition d'un signal de sortie (128) correspondant, dans lequel la courbe de variation de valeurs mesurées (125) indique la variation temporelle de valeurs mesurées (122) et un déploiement de courbe de variation (123) de la variation des valeurs mesurées (122), et dans lequel, par son emplacement et sa structure dans le diagramme (124) à délivrer en sortie, la courbe de variation de valeurs mesurées (125) indexe la variation temporelle de valeurs mesurées (122) de la valeur mesurée médicale (107), et dans lequel le déploiement de courbe de variation (123) associé respectivement à un point temporel de la courbe de variation de valeurs mesurées (125) est une mesure de la qualité de signal présente à ce point temporel de la courbe de variation de valeurs mesurées (125) en fonction des données de qualité de signal (116) ; et
- réception du signal de sortie (128) et sortie graphique du diagramme (124) à délivrer en sortie comportant la courbe de variation de valeurs mesurées (125), dans lequel le déploiement de courbe de variation (123) est représenté comme déploiement (126) correspondant de la variation temporelle de valeurs mesurées (122).

14. Programme informatique comportant un code de programme pour la mise en oeuvre d'un procédé (800) selon la revendication 13, lorsque le code de programme est exécuté sur un ordinateur, un processeur ou un composant matériel programmable.
